# EUROPEAN PATENT APPLICATION

(11) **EP 4 302 785 A1**
(43) Date of publication of application: **10.01.2024**
(21) Application number: 22761939.2
(22) Date of filing: 02.03.2022
(51) Int. Cl.: A61K 48/00, A61K 35/76, A61K 35/761, A61K 38/22, A61P 1/18, A61P 3/10, A61P 43/00, C07K 14/475, C07K 14/485, C07K 14/62, C12N 5/10, C12N 15/12, C12N 15/18, C12N 15/19, C12N 15/86, C12N 15/861, C12N 15/864

(54) **HB-EGF GENE THERAPY FOR DIABETES**

(30) Priority: 03.03.2021 JP 2021033714; 07.09.2021 JP 2021145796
(71) Applicant: Kagoshima University, Kagoshima-shi, Kagoshima 890-8580 (JP)
(72) Inventor: KOSAI, Ken-ichiro, Kagoshima-shi, Kagoshima 890-8580 (JP); MATSUDA, Eriko, Kagoshima-shi, Kagoshima 890-8580 (JP)
(74) Representative: Weickmann & Weickmann PartmbB
(86) International application number: PCT/JP2022/008898
(87) International publication number: WO 2022/186282

(57) **Abstract**

The present invention provides a novel therapeutic means that is relatively low invasive and capable of exerting a desired therapeutic effect on diabetes including T1D. Specifically, an agent for protecting and/or regenerating a pancreatic β cell in a mammal with diabetes is provided, which contains a nucleic acid encoding a heparin-binding epidermal growth factor-like growth factor (HB-EGF), wherein the agent is systemically administered. The aforementioned preparation in combination with a nucleic acid encoding hepatocyte growth factor (HGF) is also provided.

## Description

### [Technical Field]

The present invention relates to a gene therapeutic agent for diabetes whose therapeutic effect is ensured. More particularly, the present invention relates to an agent for protecting and/or regenerating pancreatic β cells in a mammal with diabetes comprising a nucleic acid encoding heparin-binding EGF-like growth factor (HB-EGF), and the like.

### [Background Art]

According to a report by the International Diabetes Federation (IDF), the number of diabetes patients in the world has reached 463 million as of 2019, and is predicted to reach 700 million by 2045 if the number continues to grow at this rate. In type 2 diabetes (T2D) accounting for 90% of all diabetes, when insulin sensitivity decreases due to genetic predisposition and environmental factors (lifestyle), pancreatic β cells try to compensate for the lack of insulin action by compensatory hypersecretion of insulin. If this state continues for a long time, the β cells become exhausted, and cannot secrete a sufficient amount of insulin, resulting in hyperglycemia.

On the other hand, type 1 diabetes (T1D) is a disease in which insulin secretion is depleted due to destruction of pancreatic β cells by an autoimmune mechanism, leading to hyperglycemia. T1D develops at a young age and the details of its mechanism are still unclear.

T1D patients need to self-inject insulin for the rest of their lives to control their blood sugar. However, glycemic control is difficult even when strict insulin therapy is performed, and hyperglycemia and severe hypoglycemia recur, making it difficult to prevent progress of complications. Therefore, an innovative therapeutic method that can completely cure T1D is demanded.

As a therapeutic method that completely cures T1D, expectations are rising for regenerative medicine that regenerates and reconstructs lost organ functions. One of them is β cell supplementation therapy by pancreatic islet transplantation, and this treatment method has been reported to be effective in improving glucose metabolism. However, since T1D patients must use immunosuppressants after pancreatic islet transplantation and there is a shortage of donors for pancreatic islet transplantation, the practical application of this technique is limited. In addition, since sustained autoimmune response against β cells occurs in T1D, it is necessary to repeat transplantation many times, even if a donor is found.

As another approach of regenerative medicine for T1D, therefore, attempts have been made to directly "protect (anti-cell death induction) and/or proliferate (regenerate) (regenerative healing induction)" (hereinafter sometimes to be comprehensively referred to as "protecting and/or regenerating") the patient's own remaining β cells in the body by in vivo gene therapy. For example, some studies using hepatocyte growth factor (HGF) as a therapeutic gene for T1D have been reported. The present inventors have recently developed a HGF gene therapeutic agent that can be administered in a low dose and exerts effect(s) protecting and/or regenerating pancreatic β cells, in view of recent reports on three dead cases due to serious adverse events in clinical trials of gene therapy using a high dose of adeno-associated virus (AAV) vector, and filed a patent application (Patent Literature 1).

Heparin-binding epidermal growth factor-like growth factor (HB-EGF) is a growth factor belonging to the EGF family. It is known that it is first synthesized as a membrane-binding type precursor (proHB-EGF), cleaved by a specific metalloproteinase at the juxtamembrane domain, and the resulting soluble HB-EGF shows a mitogenic action on many types of cells. However, HB-EGF gene therapy for T1D has been hardly studied. Only, it has been reported that results of intraperitoneal glucose tolerance test (IPGTT) in T1D model mice with alloxan partial pancreatic perfusion to which adenoviral (Ad) vector encoding HB-EGF was topically administered through retrograde pancreatic duct injection suggested that newly differentiated or proliferated insulin-producing cells could secrete insulin in response to glucose stimulation (on Patent Literature 1). However, since the blood glucose at 0 min (fasting blood glucose) in IPGTT showed a normal value even in non-treated group and no significant difference was observed between HB-EGF administered group and non-treated group, this animal model did not show T1D pathology. Furthermore, since hyperglycemia could not been suppressed even after 30 min, when insulin secretion level is supposed to increase to the maximum, it is interpreted that glucose responsive insulin secretion was not improved for exerting a therapeutic effect on T1D. In addition, this administration method is unrealistic for clinical application to human due to its high invasiveness and complicated manipulations.

### [Citation List]

### [Patent Literature]

[PTL 1]
Japanese Patent Application No. 2020-192844

### [Non Patent Literature]

[NPL 1]
Kozawa, J. et al., Pancreas, 31: 32-42 (2005)

### [Summary of Invention]

### [Technical Problem]

Therefore, the object of the present invention is to provide a novel therapeutic means that is relatively low invasive and capable of exerting a desired therapeutic effect on diabetes including T1D.

### [Solution to Problem]

To achieve the above-mentioned object, the present inventors conducted intensive studies. As a result, when HB-EGF gene was administered to T1D model mice by systemic administration rather than by topical administration to pancreas, in the mice, elevation of blood glucose was suppressed over a long term of at least 10 weeks after administration. Furthermore, elevation of blood glucose was suppressed after glucose administration in IPGTT, also in insulin secretion reaction, it was confirmed that the secretion level increased after glucose administration. Coadministration of HGF gene with HB-EGF gene confers a synergistic effect, and hyperglycemia was significantly suppressed for a long term. Also, in IPGTT, elevation of blood glucose was significantly suppressed, and insulin secretion level was significantly maintained. In the below-mentioned Examples, despite of use of an Ad vector in which a transgene is transiently expressed (typically 2-3 weeks), surprisingly, the present inventors succeeded in sustained hyperglycemia suppressing effect and glucose tolerance improving effect, far beyond predicted expression period.

In the meantime, in the below-mentioned Examples, no adverse event due to vector administration of Ad vector was observed during the test including the subsequent observation period.

Based on these findings, the present inventors succeeded in providing a novel gene therapy effective for diabetes including T1D, wherein the systemic administration (e.g., intravenous administration) of HB-EGF gene, preferably further HGF gene, which is low invasive and allows protection and/or regeneration of β cells retaining their normal function, which resulted in the completion of the present invention.

Accordingly, the present invention is as follows.

### [Item 1]

An agent for protecting and/or regenerating a pancreatic β cell in a mammal with diabetes, comprising a nucleic acid encoding a heparin-binding epidermal growth factor-like growth factor (HB-EGF), wherein the agent is systemically administered.

### [Item 2]

The agent according to item 1, in combination with a nucleic acid encoding hepatocyte growth factor (HGF).

### [Item 3]

The agent according to item 1 or 2, wherein the pancreatic β cell retains glucose-responsive insulin secretory capacity.

### [Item 4]

The agent according to any one of items 1 to 3, wherein the systemic administration is intravenous administration.

### [Item 5]

The agent according to any one of items 1 to 4, wherein the nucleic acid is carried on a viral vector.

### [Item 6]

The agent according to item 5, wherein the viral vector is an adenovirus (Ad) vector or an adeno-associated virus (AAV) vector.

### [Item 7]

The agent according to item 6, wherein the agent is administered in a single dose or administered in multiple doses with at least 60 days interval.

### [Item 8]

The agent according to any one of items 5 to 7, wherein the viral vector is administered at a dose of 1×10¹⁰ to 2×10¹² viral particles (vp)/kg body weight.

### [Item 9]

The agent according to any one of items 1 to 8, wherein the diabetes is type 1 diabetes.

### [Item 10]

The agent according to any one of items 1 to 9, wherein the mammal is human.

### [Item 11]

A method of protecting and/or regenerating a pancreatic β cell in a mammal with diabetes comprising systemically administering to the mammal an effective amount of a nucleic acid encoding HB-EGF.

### [Item 12]

The method according to item 11, further comprising administering to the mammal an effective amount of a nucleic acid encoding HGF.

### [Advantageous Effects of Invention]

According to the present invention, the desired effect of protecting and/or regenerating pancreatic β cells can be achieved over a long period of time even with safety and low invasiveness. Accordingly, in vivo high QOL gene therapy for diabetes that requires insulin administration, including T1D, becomes possible, and the realizability of the application of this treatment to human clinical practice can be enhanced.

### [Brief Description of Drawings]

[Fig. 1]

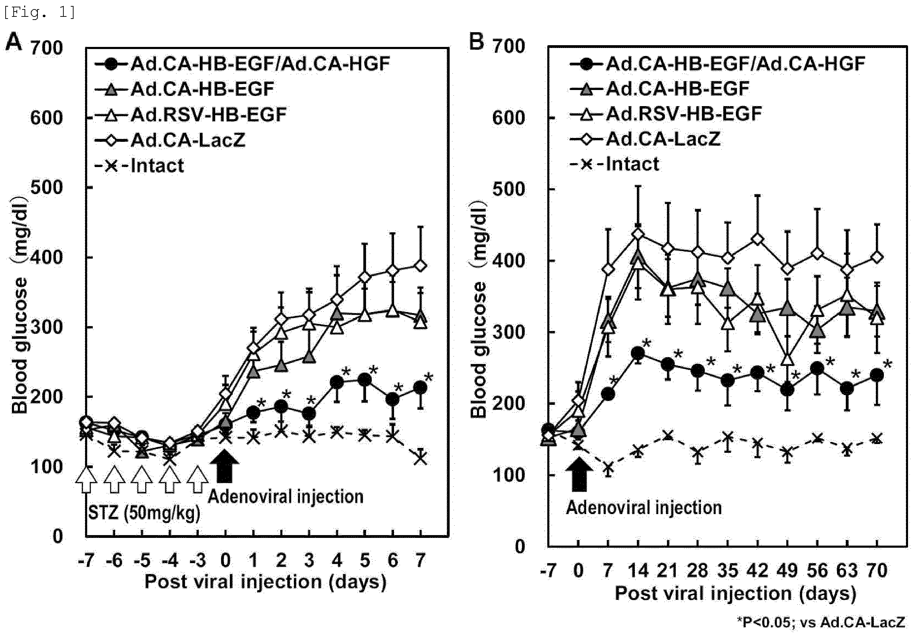

   Fig. 1A shows daily change in blood glucose level between day -7 to day 7 when the adenoviral vector was injected from the tail vein to mice that developed T1D by streptozotocin (STZ) administration. The day when the adenoviral vector was administered is Day 0. Fig. 1B shows weekly change in blood glucose level between day -7 to day 70 in the aforementioned T1D model mice administered with the adenoviral vector. In the figures, the horizontal axis indicates the number of days (days) after viral vector administration, and the vertical axis indicates the blood glucose level (mg/dl).
[Fig. 2]

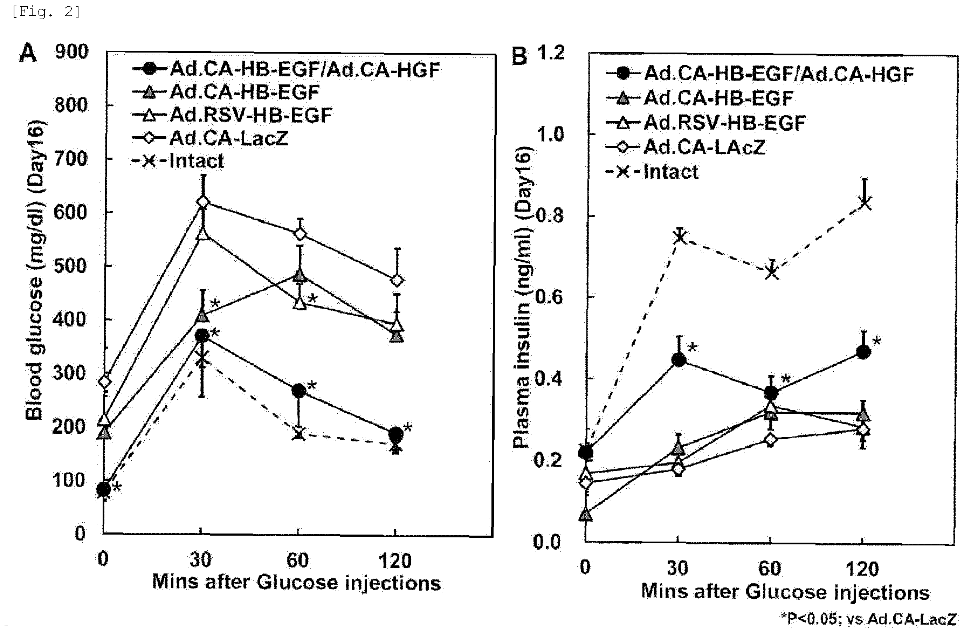

   Fig. 2 shows the results of IPGTT in the same mice as in Fig. 1 (on 16 days after Ad vector administration). Blood was collected at the time when glucose (2 g/kg body weight) was administered, 30, 60 and 120 min after administration, and blood glucose level and plasma insulin level were measured. Fig. 2A shows time-course changes in the blood glucose level (mg/dl) after glucose administration, and Fig. 2B shows time-course changes in the plasma insulin level (ng/ml) after glucose administration.
[Fig. 3]

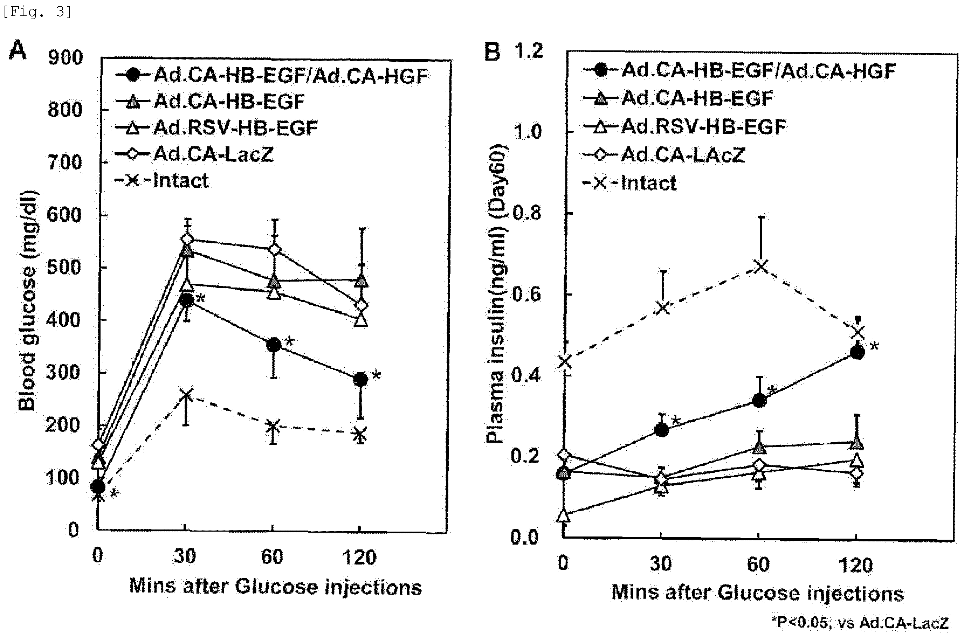

   Fig. 3 shows the results of IPGTT in the same mice as in Fig. 1 (60 days after Ad vector administration). Blood was collected at the time when glucose (2 g/kg body weight) was administered, 30, 60 and 120 min after administration, and blood glucose level and plasma insulin level were measured. Fig. 3A shows time-course changes in the blood glucose level (mg/dl) after glucose administration, and Fig. 3B shows time-course changes in the plasma insulin level (ng/ml) after glucose administration.
[Fig. 4]

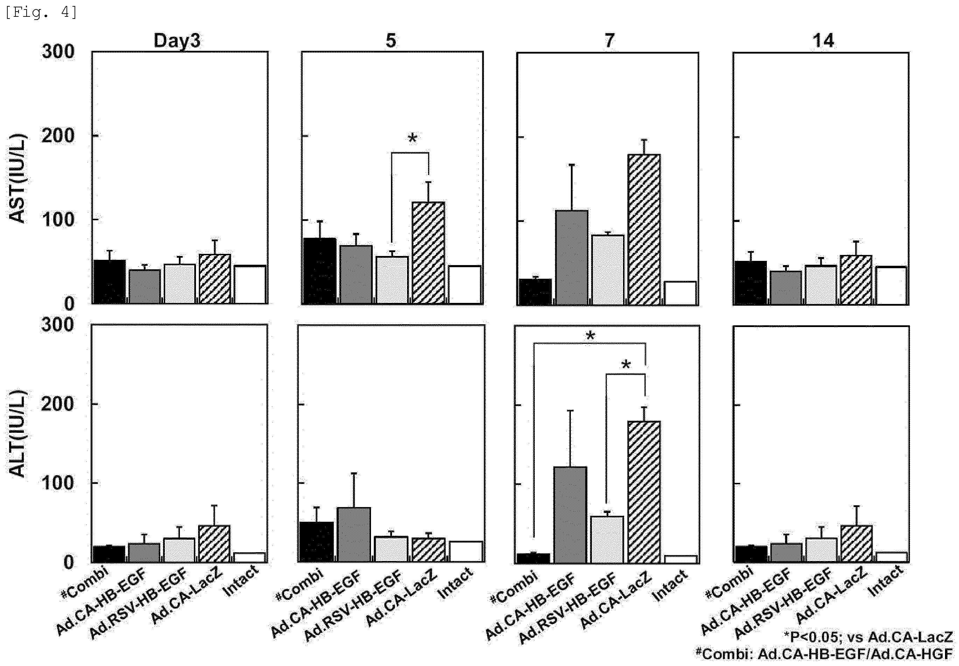

   Fig. 4 shows the results of biochemical analyses in which plasma AST levels and plasma ALT levels on day 3, day 5, day 7, and day 14 in the same 5 groups of mice as in Fig. 1 were measured. The vertical axis indicates plasma AST level (IU/L) or plasma ALT level (IU/L).
[Fig. 5]

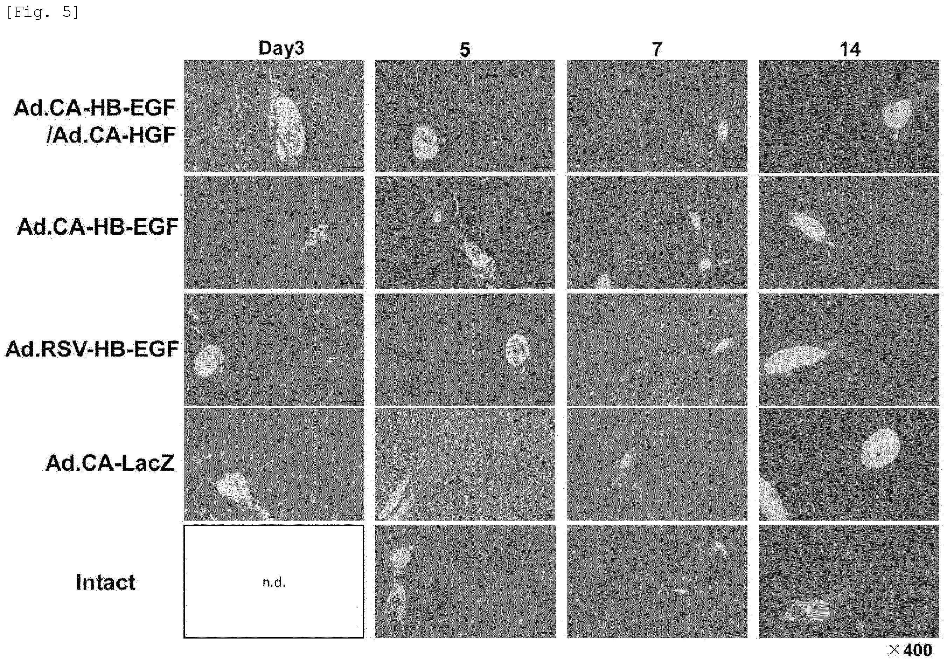

   Fig. 5 shows histological analysis (HE staining) using samples (liver) after viral administration.
[Fig. 6]

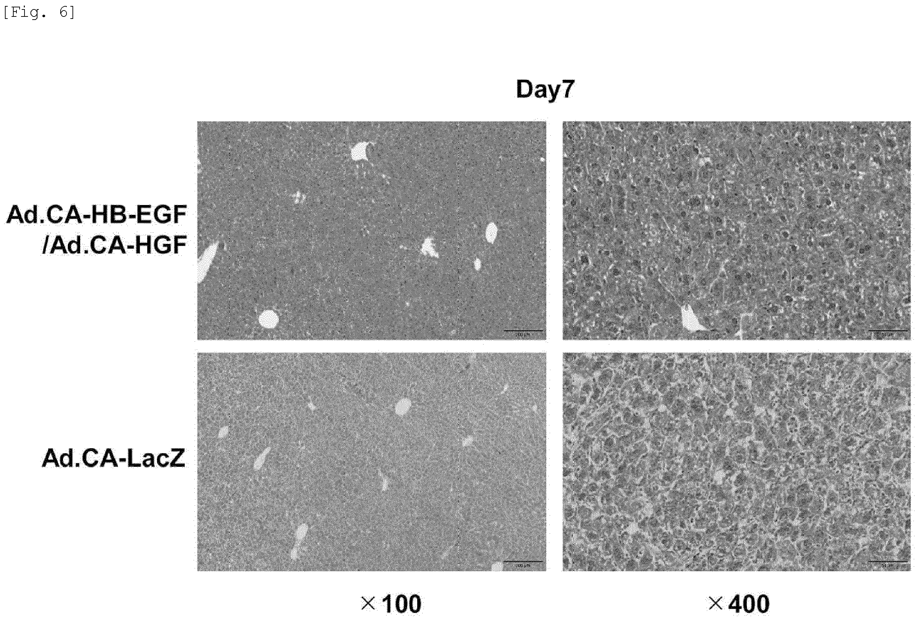

   Fig. 6 shows histological analysis (HE staining) using samples (liver) from mice administered with Ad.CA-HB-EGF and Ad.CA-HGF and mice administered with Ad.CA-LacZ.

### [Description of Embodiments]

### 1. Agent for protecting and/or regenerating β cell (I) of the present invention

The present invention provides an agent for protecting and/or regenerating a pancreatic β cell in a mammal with diabetes, comprising a nucleic acid encoding HB-EGF (hereinafter also to be referred to as "agent for protecting and/or regenerating β cell (I) of the present invention"). The agent for protecting and/or regenerating β cell is characterized in that it is administered to a target mammal by systemic administration.

In the present specification, "protecting and/or regenerating pancreatic β cells" means that the functions of the remaining β cells are retained and/or β cells are proliferated (encompassing both self-regeneration of existing β cells and differentiation from stem cell or endocrine progenitor cell) to the extent that at least the acute phase of hyperglycemia is significantly suppressed as compared with the control without a therapeutic treatment, and the suppression tendency is maintained over a long term thereafter (e.g., not less than 60 days, preferably not less than 75 days, more preferably not less than 90 days, further preferably not less than 120 days), without causing compensatory hypersecretion of insulin from β cells and while maintaining normal insulin secretion. The "protecting and/or regenerating a pancreatic β cell" in the present invention inevitably involves "suppression of hyperglycemia", and thus, "the agent for protecting and/or regenerating β cell of the present invention" can also be an "agent for suppressing hyperglycemia". In addition, since suppressing hyperglycemia and controlling blood glucose is most important in treating diabetes and suppressing progression to complications, the "agent for protecting and/or regenerating β cell of the present invention" is also an "agent for treating diabetes". According to the above-mentioned Non Patent Literature 1, when HB-EGF gene is topically administered to mice treated by alloxan partial pancreatic perfusion using an Ad vector through retrograde pancreatic duct injection, it has been reported that no significant difference in fasting blood glucose was observed as compared with non-treated (LacZ gene-administered) group. Since the fasting blood glucose in non-treated group was a normal value, it is suggested that the animal model used never shows T1D pathology (β cells capable of secreting insulin are maintained to the extent that fasting hyperglycemia can be suppressed). Accordingly, the prior art reference fails to demonstrate a therapeutic effect on T1D. On the other hand, the present invention demonstrates, in the below-mentioned Examples, that fasting blood glucose (blood glucose at 0 min in IPGTT) in non-treated group shows a value remarkably higher than that of normal mice (namely, the animal model clearly shows T1D pathology), and that fasting blood glucose in HB-EGF gene-administered group is reduced as compared with non-treated group. Therefore, the agent for protecting and/or regenerating β cell (I) of the present invention, which can suppress fasting hyperglycemia, exerts an advantageous effect against the known HB-EGF gene therapeutic agent.

Preferably, "protection and/or regeneration of a pancreatic β cell" means protection and/or regeneration of β cell that retains a function secreting insulin in response to glucose stimulation and suppressing elevation of blood glucose by glucose loading, namely, glucose-responsive insulin secretory capacity. Even if the amount of β cells is retained by protection of the residual β cells and/or differentiation of proliferation of new β cells, when glucose-responsive insulin secretory capacity of β cells is low, postprandial hyperglycemia may not be suppressed and sufficient blood glucose control may not be achieved. For example, in the above-mentioned Non Patent Literature 1, mice treated by alloxan partial pancreatic perfusion tested are considered to retain β cells capable of secreting insulin to the extent that fasting blood glucose is maintained to a normal level, nonetheless, no significant difference in the change in blood glucose was observed between HB-EGF gene-administered group and non-treated group until at least 60 min after glucose loading, which shows that pancreatic topical administration of HB-EGF gene does not exert a sufficient suppressing effect on postprandial hyperglycemia. On the other hand, it is demonstrated that the agent for protecting and/or regenerating β cell (I) of the present invention exerts a glucose-responsive insulin secretory effect and postprandial hyperglycemia-suppressing effect based thereon in IPGTT, and it retains such effects for a long period of time.

The "nucleic acid encoding HB-EGF" to be used in the present invention can be DNA or RNA, or DNA/RNA chimera. Preferably, DNA can be mentioned. Also, the nucleic acid can be double-stranded or single-stranded. When it is double-stranded, it can be double-stranded DNA, double-stranded RNA, or DNA:RNA hybrid. When it is single stranded, it can be sense strand (i.e., coding strand) or antisense strand (i.e., noncoding strand). As the DNA encoding HB-EGF, genomic DNA, cDNA (cRNA) derived from human or other mammalian cells or tissues, synthetic DNA(RNA), and the like can be mentioned.

The "nucleic acid encoding HB-EGF" used in the present invention includes at least a nucleotide sequence encoding a secretion type soluble HB-EGF. Preferably, the "nucleic acid encoding HB-EGF" includes a nucleotide sequence encoding proHB-EGF, which is a membrane-bound HB-EGF, and more preferably includes a nucleotide sequence encoding preproHB-EGF, which is an initial translation product containing a signal sequence at the N-terminal. HB-EGF is first synthesized as a precursor containing a signal sequence, after which the signal sequence is cleaved in the endoplasmic reticulum to form membrane-bound proHB-EGF, which is further cleaved by a specific metalloproteinase in the juxtamembrane domain to become soluble HB-EGF. In the present invention, a nucleic acid encoding HB-EGF that has entered the bloodstream by systemic administration is delivered to the organ targeted by the nucleic acid or vector (e.g., viral vector) such as the liver. When the targeted organ is other than the pancreas, the expressed and secreted soluble HB-EGF from the cells of the organ is considered to be delivered to the pancreas via the bloodstream, and mainly exert a protective and/or regenerating action on β cells. Therefore, as long as at least a nucleotide sequence encoding soluble HB-EGF is included, it can be directly expressed and secreted as soluble HB-EGF by inserting same into a secretory expression vector containing an appropriate signal sequence. However, since there is a possibility that the artificial secretory expression mechanism does not function well, it is preferable to first express membrane-bound proHB-EGF, and then to generate soluble HB-EGF by utilizing the action of protease endogenous to the administration subject mammal. Although the secretion efficiency can be improved by substituting the signal sequence with a sequence other than the native signal sequence, the secretion efficiency may also be decreased. In a preferred embodiment, therefore, a nucleic acid encoding preproHB-EGF including the native signal sequence can be used.

More specifically, as the "nucleic acid encoding HB-EGF", a nucleic acid containing a nucleotide sequence that hybridizes under stringent conditions with a complementary strand sequence of the nucleotide sequence shown by SEQ ID NO:1 [corresponding to nucleotide sequence (CDS) from positions 276 to 902 of human HB-EGF mRNA sequence registered in GenBank under Accession Number: NM_001945 (276th to 332nd correspond to signal codon, 333rd to 461st correspond to propeptide coding region, and 462nd to 719th correspond to soluble HB-EGF coding region)], and that encodes a protein having activity (e.g., pancreatic β cell protecting and/or regenerating activity) equivalent to that of HB-EGF can be mentioned.

Examples of the nucleic acid that hybridizes to the complementary strand sequence of the nucleotide sequence shown by SEQ ID NO:1 (provided that when the nucleic acid is RNA, "t" in the base sequence is replaced with "u") under stringent conditions include a nucleic acid containing a nucleotide sequence showing an identity of about 60% or more, preferably about 70% or more, more preferably about 80% or more, and particularly preferably about 90% or more, most preferably about 95% or more, with the nucleotide sequence shown by SEQ ID NO:1, and the like.

The nucleic acid encodes an amino acid sequence showing an identity of about 90% or more, preferably about 95% or more, further preferably about 97% or more, and particularly preferably about 98% or more, with the amino acid sequence shown by SEQ ID NO:2, such that a protein containing the amino acid sequence has substantially the same activity (e.g., pancreatic β cell protecting and/or regenerating activity) as a protein containing the amino acid sequence shown by SEQ ID NO:2.

The identity of the base sequence in the present specification can be calculated using homology calculation algorithm NCBI BLAST (National Center for Biotechnology Information Basic Local Alignment Search Tool) under the following conditions (expectancy=10; allowing gap; filtering=ON; match score=1; mismatch score=-3).

The identity of the amino acid sequence in the present specification can be calculated using homology calculation algorithm NCBI BLAST under the following conditions (expectancy=10; allowing gap; matrix=BLOSUM62; filtering=OFF).

Hybridization can be conducted according to a method known per se or a method based thereon, for example, a method described in Molecular Cloning, 2nd edition (J. Sambrook et al., Cold Spring Harbor Lab. Press, 1989) and the like. When a commercially available library is used, hybridization can be conducted according to the method described in the instruction manual attached thereto. Hybridization can preferably be conducted under stringent conditions.

The stringent conditions are exemplified by reaction conditions characterized in that (1) a low ionic strength and a high temperature, for example, 0.015 M sodium chloride/0.0015 M sodium citrate/0.1% dodecyl sodium sulfate at 50°C, is used for washing, and (2) a denaturing agent such as formamide, for example, 50% (v/v) formamide along with a 50 mM sodium phosphate buffer (pH 6.5) containing 0.1% bovine serum albumin/0.1% Ficoll/0.1% polyvinylpyrrolidone/750 mM sodium chloride and 75 mM sodium citrate is used at 42°C. Alternatively, the stringent condition can be a condition in which 50% formamide, 5xSSC (0.75 M sodium chloride, 0.075 M sodium citrate), 50 mM sodium phosphate (pH 6.8), 0.1% sodium pyrophosphate, 5x Denhart's solution, sonicated salmon sperm DNA (50 µg/ml), 0.1% SDS, and 10% dextran sulfate are used at 42°C, and a washing is performed with 0.2xSSC and 50% formaldehyde at 55°C, followed by a high-stringent washing comprised of EDTA-containing 0.1xSSC at 55°C. Those of ordinary skill in the art can easily achieve a desired stringency by appropriately adjusting temperature at hybridization reaction and/or washing, ion strength of buffer, and the like based on factors such as probe length.

The nucleic acid encoding HB-EGF may be an ortholog, in non-human mammals, of the nucleic acid consisting of the nucleotide sequence shown by SEQ ID NO:1. For example, it is desirable to use a nucleic acid encoding HB-EGF derived from a mammal as a subject of administration. The mammal to be the subject of administration of the agent for protecting and/or regenerating β cells of the present invention is not particularly limited as long as it has diabetes, and includes human, mouse, rat, rabbit, dog, monkey, and the like, preferably human. Therefore, in a preferred embodiment, the nucleic acid encoding HB-EGF is a nucleic acid encoding human HB-EGF (i.e., a protein consisting of the amino acid sequence shown by SEQ ID NO:2).

The nucleic acid encoding HB-EGF can be cloned by, for example, amplifying it by the PCR method using a synthetic DNA primer having a portion of the nucleotide sequence of the CDS region of the HB-EGF gene, or by hybridizing DNA incorporated in an appropriate expression vector to a labeled DNA fragment or synthetic DNA containing the nucleotide sequence of the CDS region of the HB-EGF gene. Hybridization can be conducted according to, for example, a method described in Molecular Cloning, 2nd edition (ibidem), and the like. When a commercially available library is used, hybridization can be conducted according to the method described in the instruction manual attached to the library.

The nucleotide sequence of DNA can be converted according to a method known per se, such as the ODA-LA PCR method, the Gapped duplex method, the Kunkel method and the like, or a method based thereon, using a publicly known kit, for example, Mutan^{™}-super Express Km (Takara Shuzo Co., Ltd.), Mutan^{™}-K (Takara Shuzo Co., Ltd.) and the like.

The cloned DNA can be used as is, or after digestion with a restriction endonuclease or addition of a linker as desired, depending on the purpose of its use. The DNA may have the translation initiation codon ATG at the 5' end thereof, and the translation stop codon TAA, TGA or TAG at the 3' end thereof. These translation initiation codons and translation stop codons can be added using an appropriate synthetic DNA adapter.

An expression vector containing a nucleic acid encoding HB-EGF can be produced by, for example, cutting out a desired DNA fragment from the nucleic acid encoding the CDS region of the HB-EGF gene, and joining the DNA fragment downstream of a promoter in an appropriate expression vector.

The expression vector is not particularly limited as long as it is generally used for gene therapy. For example, viral vectors such as adenovirus (Ad) vector, adeno-associated virus (AAV) vector, lentiviral vector, retroviral vector, sindbis viral vector, rabies viral vector, Sendaiviral vector, simple herpes viral vector, and non-viral vectors such as animal cell expression plasmid (e.g., pA1-11, pXT1, pRc/CMV, pRc/RSV, pcDNAI/Neo) and the like can be used. It is preferable to use an Ad vector or an AAV vector from the aspects of high gene transfer-expression efficiency, low frequency of chromosomal integration and no risk of insertional mutation, ability of introduction into nondividing cells, ability of medium- to long-term expression of transgene, and the like.

The transgene expression period of Ad vector (generally 2-3 weeks) is shorter than that of AAV vectors and chromosomal-integrating vectors. However, since the pancreatic β cells protection and/or regeneration effect by HB-EGF persists far beyond the gene expression period (at least 60 days or more, preferably 75 days or more, more preferably 90 days or more, further preferably 120 days or more), it may be rather advantageous in that the risk of side effects due to long-term expression of HB-EGF such as carcinogenesis can be reduced or avoided. Although the size of the gene that the AAV vector can carry is as small as 4.7 kb, since the HB-EGF coding sequence (CDS) is 624 kb, and the entire expression cassette including the promoter, terminator, and so on is about 1 to 2 kb, there is no problem in use thereof.

Ad vector is known to accumulate in the liver. Even if HB-EGF is introduced and expressed in the cells of other organs, extracellularly secreted soluble HB-EGF may be delivered to the pancreas through the bloodstream. In addition, AAV vectors have different tissue tropism depending on the serotype, and serotypes having tropism toward the pancreas include, for example, types 6, 8, and 9. However, the serotype to be used is not particularly limited, since soluble HB-EGF introduced, expressed and secreted in the cells of other organs can still be delivered to the pancreas through the bloodstream when a ubiquitous promoter is used. Rather, in some cases, it may be preferable to express HB-EGF in the cells of other organs because, when viral vectors accumulate in the pancreas, β cells may be attacked by viral capsid antigen-specific killer T cells.

The promoter may be any promoter, as long as it is appropriate for the host used to express the gene.

For example, when it is a mammalian animal cell, usable promoters include virus promoter such as promoters derived from cytomegalovirus (CMV) (e.g., CMV immediate-early promoter), promoters derived from human immunodeficiency virus (HIV) (e.g., HIV LTR), promoters derived from Rous sarcoma virus (RSV) (e.g., RSV LTR), promoters derived from mouse mammary cancer virus (MMTV) (e.g., MMTV LTR), promoters derived from Moloney murine leukemia virus (MoMLV) (e.g., MoMLV LTR), promoters derived from herpes simplex virus (HSV) (e.g., HSV thymidine kinase (TK) promoter), promoters derived from simian virus 40 (SV40) (e.g., SV40 early promoter), promoters derived from Epstein-Barr virus (EBV), promoters derived from adeno-associated virus (AAV) (e.g., AAV p5 promoter), promoters derived from adenovirus (AdV) (Ad2 or Ad5 major late promoter) and the like, and constitutive protein gene promoters of mammals such as β-actin gene promoter, phosphoglycerate kinase (PGK) gene promoter, transferrin gene promoter, and the like.

When a viral vector is used as a vector, administration at low doses is desired to reduce the risk of adverse events. However, in order to afford a desired treatment effect at a low dose, it is necessary to load a nucleic acid encoding HB-EGF at the downstream of a promoter with transcriptional activity that can achieve therapeutically effective blood HB-EGF levels. For example, a promoter with stronger transcriptional activity than the CMV promoter and RSV promoter that are frequently used in gene therapy with viral vectors can be used. As such high active promoter, CA promoters and promoters with transcriptional activity equivalent thereto, for example, ubiquitous promoters such as polypeptide chain elongation factor 1α1 (EF1A) promoter, polypeptide chain elongation factor 1α1 short (EFS) promoter, CBh promoter (hybrid promoter of CMV immediate-early enhancer and modified chicken β-actin promoter different CA promoter), spleen focus-forming virus (SFFV) promoter, mouse stem cell virus (MSCV) promoter, SV40 enhancer/early promoter, PGK promoter, ubiquitin C (UBC) promoter, and the like can be mentioned.

In addition, when a high viral vector with high tissue tropism is used, or in the case of topical administration, a promoter which is specifically and highly expressed in tissue or cells of the target organ (e.g., in liver; albumin promoter, α-fetoprotein promoter, thyroxine-binding globulin promoter, and the like, in pancreatic β cells, insulin promoter, Pdx1 promoter, Ins2 promoter, and the like, in muscle; myogenin promoter, skeletal muscle actin α1 (ACTA1) promoter, MHCK7 promoter, SM22a promoter, and the like can be unlimitatively mentioned, including promoters specific for tissues or cells of any organ from which the expressed and secreted HB-EGF can be delivered to the pancreas by the bloodstream) can also be used.

The expression vector preferably contains a transcription termination signal, i.e., terminator region, in the downstream of the nucleic acid encoding HB-EGF. In addition, when desired, it may further contain enhancer, splicing signal, selection marker gene for selection of transformed cells, SV40 replication origin, and the like. Examples of the selection marker gene include genes that offer resistance against agents such as tetracycline, ampicillin, kanamycin, hygromycin, and phosphinothricin, genes that complement an auxotrophic mutation, and the like, and the like.

Where necessary, a nucleotide sequence encoding a signal sequence (signal codon) suitable for the host can be added to the 5'-end side of the DNA encoding HB-EGF. For example, insulin signal sequence, α-interferon signal sequence, antibody molecule signal sequence, and the like can be used.

An expression vector containing a nucleic acid encoding HB-EGF can be produced using a conventional genetic engineering technique, cell culturing technique, and virus preparation technique [for example, Current Protocols in Molecular Biology, F. Ausubel et al., eds. (1994) John Wiley & Sons, Inc.; Molecular Cloning (A Laboratory Manual), 3rd ed. Volumes 1-3, Josseph Sambrook & David W. Russel eds., Cold Spring Harbor Laboratory Press (Cold Spring Harbor, New York) (2001); Culture of Animal Cells; A Manual of Basic Technique, R. Freshney eds., 2nd ed. (1987), Wiley-Liss; Frank L. Graham, Manipulation of adenoviral vector, Chapter 11. p109-p128; E.J. Murray eds., Methods in Molecular Biology, Vol. 7, Gene Transfer and Expression Protocols (1991); Chen, S-H. et al., Combination gene therapy for liver metastases of colon carcinoma in vivo., Proc. Natl. Acad. Sci. USA (1995) 92, 2477-2581, and the like].

When a nonviral vector is used as the expression vector containing a nucleic acid encoding HB-EGF, introduction of the expression vector can be performed by using a polymer carrier such as poly-L-lysine-nucleic acid complex, or by encapsulating the vector in a liposome. The liposome is a capsule composed of phospholipid with a particle size of several tens to several hundreds nm, inside of which vectors such as a plasmid encoding HB-EGF can be encapsulated.

The agent for protecting and/or regenerating β cells (I) of the present invention can suppress hyperglycemia over a long period of time while maintaining normal insulin secretion. Therefore, it can be used for the treatment of T1D and other diabetes (e.g., T2D where insulin resistance progresses and β cells are exhausted due to compensatory insulin hypersecretion, resulting in impaired insulin secretion and eventual β cell death) requiring insulin administration due to the destruction of pancreatic β cells, as well as for the suppression of the progression into complications.

In the agent for protecting and/or regenerating β cells (I) of the present invention, the HB-EGF-expressing viral vector of the present invention may be used as it is, or may be, where necessary, mixed with a pharmacologically acceptable carrier and formulated into various forms of preparation such as injection and the like, and used as a medicament.

Here, as examples of the pharmacologically acceptable carrier, various organic or inorganic carrier substances conventionally used as pharmaceutical preparation materials can be mentioned, and in liquid preparations, these are formulated as solvents, solubilizing agents, suspending agents, isotonizing agents, buffering agents and soothing agents, and the like. Also, as necessary, pharmaceutical preparation additives such as antiseptics, antioxidants, colorants, and the like can be used.

As examples of suitable solvents, water for injection, physiological saline, Ringer's solutions, alcohols, propylene glycol, polyethylene glycol, sesame oil, corn oil, olive oil, cottonseed oil and the like can be mentioned.

As examples of suitable solubilizing agents, polyethylene glycol, propylene glycol, D-mannitol, trehalose, benzyl benzoate, ethanol, trisaminomethane, cholesterol, triethanolamine, sodium carbonate, sodium citrate, sodium salicylate, sodium acetate and the like can be mentioned.

As examples of suitable suspending agents, surfactants such as stearyl triethanolamine, sodium lauryl sulfate, lauryl aminopropionic acid, lecithin, benzalkonium chloride, benzethonium chloride and glyceryl monostearate; hydrophilic polymers such as poly(vinyl alcohol), polyvinyl pyrrolidone, sodium carboxymethyl cellulose, methyl cellulose, hydroxymethyl cellulose, hydroxyethyl cellulose and hydroxypropyl cellulose; polysorbates, polyoxyethylene hardened castor oil and the like can be mentioned.

As examples of suitable isotonizing agents, sodium chloride, glycerin, D-mannitol, D-sorbitol, glucose and the like can be mentioned.

As examples of suitable buffers, buffer solutions of a phosphate, an acetate, a carbonate, a citrate and the like, and the like can be mentioned.

As examples of suitable soothing agents, benzyl alcohol and the like can be mentioned.

As examples of suitable antiseptics, paraoxybenzoates, chlorobutanol, benzyl alcohol, phenethyl alcohol, dehydroacetic acid, sorbic acid and the like can be mentioned.

As examples of suitable antioxidants, sulfides, ascorbates and the like can be mentioned.

As examples of suitable colorant s, aqueous food tar colors (e.g., food colors such as Food Red Nos. 2 and 3, Food Yellow Nos. 4 and 5, and Food Blue Nos. 1 and 2), water-insoluble lake pigments (e.g., aluminum salts of the aforementioned aqueous food tar colors and the like), natural pigments (e.g., β-carotene, chlorophyll, red iron oxide and the like) and the like can be mentioned.

As examples of dosage forms of the aforementioned pharmaceutical composition, non-oral formulations such as injections (e.g., subcutaneous injections, intravenous injections, intramuscular injections, intraperitoneal injections and the like), drops, and the like can be mentioned.

The agent for protecting and/or regenerating β cells (I) of the present invention can be produced by a method conventionally used in the field of formulation technology, such as the method described in the Japanese Pharmacopoeia. The content of the viral vector, which is the active ingredient in the preparation, varies depending on the dosage form, dose of the active ingredient, and the like and is, for example, about 0.1 to 100 wt%. The virus titer can be appropriately adjusted to be about, for example, 10¹⁰ - 10¹¹ pfu/ml (since the physical titer is several to 100 times or more higher than the biological titer, 2×10¹⁰ - 2×10¹³ vp/ml in terms of virus particles), but is not limited to this range.

Examples of preparations suitable for parenteral administration (e.g., intravenous injection, subcutaneous injection, intramuscular injection, intraperitoneal administration and the like) include aqueous and nonaqueous isotonic aseptic injection liquids, in which antioxidant, buffering agent, antiviral agent, isotonizing agent and the like can be contained. The examples also include aqueous and nonaqueous aseptic suspension liquids optionally containing suspension agent, solubilizer, thickener, stabilizer, preservative and the like. The most preferred dosage form in the present invention is an injection liquid.

Dose of the preparation varies depending on the kind of vector, promoter activity, administration route, severity of illness, the animal species to be the subject of administration, drug acceptability, body weight, age, and the like of the subject of administration. For example, when Ad vector is used as an HB-EGF expression vector, in past clinical studies, deaths due to acute liver injury have been reported after hepatic arterial administration of 6×10¹¹ viral particles (vp)/kg body weight (total amount 3.8×10¹³ vp) (Mol Genet Metab 2003; 80: 148-158). Since Ad vectors are highly accumulated in the liver, higher doses of Ad vectors are expected to accumulate in the liver in the case of hepatic artery administration. Therefore, it is assumed that Ad vectors can be safely used up to about 10 times the dose or 5×10¹² vp/kg body weight for other systemic administration, particularly when Ad vectors are administered through a peripheral vein. For example, a single dose is about 5×10⁹ - about 5×10¹² vp/kg body weight, preferably about 1×10¹⁰ - about 2×10¹² vp/kg body weight. Also, when AAv vector is used as an HB-EGF expression vector, in recent clinical studies, deaths due to serious hepatopathy have been reported in a high dose (3×10¹⁴ vp/kg body weight) administration group (Audentes Therapeutics. Letter to the MTM disease community. https://myotubulartrust.org/audentes-therapeutics-letter-23-june-2020/; Hum Gene Ther 2020; 31: 695-696). However, in the same clinical study, the low dose (1×10¹⁴ vp/kg body weight) group did not experience any adverse events in the liver despite having liver disease, suggesting that doses of 10¹⁴ vp/kg body weight or less can be used safely. For example, a single dose is about 5×10⁹ - about 5×10¹³ vp/kg body weight, preferably about 1×10¹⁰ - about 1×10¹³ vp/kg body weight.

For example, in the below-mentioned Example, a tail vein administration of Ad vector at about 5×10¹² vp/kg body weight demonstrated the effect of HB-EGF gene therapy on T1D. In the above-mentioned Non Patent Literature 1, a biological titer of 4×10⁸ pfu (about 2×10¹⁰ pfu/kg body weight) of Ad vector was administered to mice through retrograde pancreatic duct injection. Since the vp:pfu ratio varies greatly due to the virus extraction method and the like, the dose in terms of physical titer is unknown; however, it is estimated to be about 2×10¹² vp/kg body weight or higher when the vp:pfu ratio is 100 times or more higher. Retrograde pancreatic duct injection can remarkably reduce the dosage compared to systemic administration because its gene transfer is limited to the pancreas. Therefore, it is surprising that systemic administration at a dose equivalent to that of retrograde pancreatic duct injection can produce a more superior effect of suppressing elevation of blood glucose than retrograde pancreatic duct injection.

On the other hand, when a nonviral vector encapsulated in a liposome is used as the HB-EGF expression vector, since the safety was confirmed by intravenous administration of 666 µg of DNA in a clinical research using a cynomolgus with a body weight of about 4 kg, this amount can be used as a rough standard dose. For example, the single dose for an adult is about 2 to about 10 mg, preferably about 5 to about 8 mg.

The agent for protecting and/or regenerating β cells (I) of the present invention is systemically administered, for example, parenterally (e.g., intravenously, subcutaneously, intramuscularly, intraperitoneally, and the like) by injection, catheter, balloon catheter, or the like. As used herein, the " systemic administration" may be an administration method other than local administration to the pancreas, such as intravenous administration, intramuscular administration, intraperitoneal administration, and the like as long as the nucleic acid encoding HB-EGF in the administered preparation and/or the soluble HB-EGF produced and secreted from the nucleic acid enters the blood stream and circulates throughout the body, thereby contributing to the protection and/or regeneration of pancreatic β cells.

Local administration into the pancreatic duct is considered to be apparently advantageous in that the risk of adverse events due to gene transfer to multiple organs can be avoided, since gene transfer by the administration is almost confined to the pancreas. However, given the risk that β cells may be destroyed by the attack of killer T-cells against transgenes and viruses, systemic administration, which allows for gene transfer to other organs, is clinically advantageous.

When retrograde pancreatic duct injection is applied to human clinical practice, even though there is no need for laparotomy, gene transfer must be performed by endoscopic retrograde cholangiopancreatography (ERCP), which is highly invasive and requires complicated procedures. Therefore, systemic administration such as intravenous injection is extremely advantageous in terms of patient tolerance and convenience. Furthermore, according to the above-mentioned Non Patent Literature 1, retrograde pancreatic duct injection is associated with a risk of severe pancreatitis unless the vector concentration, volume, injection rate and time are strictly controlled. In this respect as well, systemic administration has higher safety.

In the above-mentioned Non Patent Literature 1, as the reasons for using gene therapy instead of injection of HB-EGF protein for HB-EGF treatment for diabetes, the authors explain that persistence of biological activity of HB-EGF protein is low and that the most part was assumed to be inactivated by proteases in pancreatic juice. They consider that the differentiation or neogenesis of β-cells from pancreatic ductal cells by the autocrine or juxtacrine action of membrane-bound proHB-EGF expressed in pancreatic ductal cells is most important, and additionally that the paracrine effect of soluble HB-EGF secreted from pancreatic ductal cells protects and/or regenerates the remaining β-cells in the pancreatic islets. Therefore, it is reasonable to conclude that a person skilled in the art, who learned the teachings of Non Patent Literature 1, could not have predicted that soluble HB-EGF expressed and secreted from remote organs such as the liver would be delivered to the pancreas by the blood flow and cause a protective and/or regenerative effect of β cells by endocrine action. Therefore, the strategy to treat diabetes by transferring the gene to organs other than the pancreas as the main target organ, protecting and regenerating β-cells by the action of soluble HB-EGF expressed and secreted from the cells of the organ, and suppressing elevation of blood glucose is novel and original.

Among the dose ranges of the viral vector used in the present invention, when a comparatively high dose is used, in order to avoid liver damage, which is particularly important as an adverse event caused by the viral vector, it is desirable to avoid administration routes such as hepatic arterial administration that deliver high doses of the viral vector to the liver, and administer the agent for protecting and/or regenerating β cells (I) of the present invention from, for example, vein, particularly peripheral vein or the like.

The administration frequency of the agent for protecting and/or regenerating β cells (I) of the present invention is not particularly limited. As shown in the following Example, even when an Ad vector is used, a single administration can afford a certain degree of hyperglycemia suppressive effect over a long period of at least 70 days. In addition, the glucose-responsive insulin secretory capacity of pancreatic β cells can be maintained (improved glucose tolerance) for at least 60 days. The mouse of Example is under ongoing progress observation, and it is fully expected that the above-mentioned effects will continue for an even longer period of time. The same may be true when an AAV vector, the transgene of which is in principle not integrated into the chromosome, is introduced into dividing cells.

In addition, when the AAV vector is used to target nondividing cells, HB-EGF expression can be maintained for a longer period of time. Therefore, it is considered that a hyperglycemia suppressive action and a glucose tolerance improving effect can be afforded for even longer period of time (e.g., 6 months or longer, preferably one year or longer, more preferably several years or longer).

Therefore, the agent for protecting and/or regenerating β cells (I) of the present invention can be administered at intervals of, for example, at least 60 days or longer, preferably 75 days or longer, more preferably 90 days or longer, and further preferably 120 days or longer, even when Ad vectors and AAV vectors, which are non-chromosomally integrated and conventionally considered safer and used more frequently than retrovirus and lentiviral vectors, are used. In addition, depending on the type of viral vector to be used, it can also be administered at an interval of once every three months to several years, and in another embodiment, single administration is also possible.

### 2. The agent for protecting and/or regenerating β cells (II) of the present invention

The present invention also provides an agent for protecting and/or regenerating pancreatic β cells in a mammal with diabetes, containing a nucleic acid encoding HB-EGF and a nucleic acid encoding HGF in combination, which is characterized in that at least the nucleic acid encoding HB-EGF is systemically administered (hereinafter also to be referred to as "the agent for protecting and/or regenerating β cells (II) of the present invention").

As the "nucleic acid encoding HGF" used in the present invention, a nucleic acid containing the nucleotide sequence shown by SEQ ID NO:3 (corresponding to nucleotide sequence (CDS) from positions 77 to 2263 of human HGF mRNA sequence registered in GenBank under Accession Number: NM_000601), or a nucleotide sequence that hybridizes to a complementary strand sequence thereof under stringent conditions, and encoding a protein having activity (e.g., pancreatic β cell protecting and/or regenerating activity) equivalent to that of HGF can be mentioned.

Examples of the nucleic acid that hybridizes to the complementary strand sequence of the nucleotide sequence shown by SEQ ID NO:3 under stringent conditions include a nucleic acid containing a nucleotide sequence showing an identity of about 60% or more, preferably about 70% or more, more preferably about 80% or more, and particularly preferably about 90% or more, most preferably about 95% or more, with the nucleotide sequence shown by SEQ ID NO:3, and the like. As used herein, the "stringent conditions" are as defined for the aforementioned nucleic acid encoding HB-EGF. The nucleic acid encodes an amino acid sequence showing an identity of about 90% or more, preferably about 95% or more, further preferably about 97% or more, and particularly preferably about 98% or more, with the amino acid sequence shown by SEQ ID NO:4, such that a protein containing the amino acid sequence has substantially the same activity (e.g., pancreatic β cell protecting and/or regenerating activity) as a protein containing the amino acid sequence shown by SEQ ID NO:4.

The nucleic acid encoding HGF may be an ortholog, in non-human mammals, of the nucleic acid consisting of the nucleotide sequence shown by SEQ ID NO:3. For example, it is desirable to use a nucleic acid encoding HGF derived from a mammal as a subject of administration. The mammal to be the subject of administration of the agent for protecting and/or regenerating β cells of the present invention is not particularly limited as long as it has diabetes, and includes human, mouse, rat, rabbit, dog, monkey, and the like, preferably human. Therefore, in a preferred embodiment, the nucleic acid encoding HGF is a nucleic acid encoding human HGF (i.e., a protein consisting of the amino acid sequence shown by SEQ ID NO:4).

The nucleic acid encoding HGF can be cloned in the same manner as the aforementioned nucleic acid encoding HB-EGF, and can be inserted downstream of the promoter of various viral vectors or non-viral vectors similar to those described above. The nucleic acid encoding HGF may be inserted into a single expression vector together with the nucleic acid encoding HB-EGF, or each may be inserted into separate expression vectors. When the nucleic acid encoding HB-EGF and the nucleic acid encoding HGF are inserted into a single expression vector, they may be placed under the control of a single promoter, or may be placed under the control of separate identical or different promoters. In the former case, either the nucleic acid encoding HB-EGF or the nucleic acid encoding HGF may be located upstream (closer to the promoter), but a sequence that enables dicistronic expression in mammalian cells (e.g., IRES sequence, foot-and-mouth disease virus-derived 2A sequence) is inserted between the both nucleic acids. Moreover, when the nucleic acid encoding HB-EGF and the nucleic acid encoding HGF are inserted into separate expression vectors, the types of expression vectors may be the same or different. Also, both nucleic acids may be placed under the control of the same or different promoters.

By using in combination with a nucleic acid encoding HB-EGF, the nucleic acid encoding HGF can suppress hyperglycemia over a long period of time while maintaining normal insulin secretion. Therefore, it can be used for the treatment of T1D and other diabetes (e.g., T2D where insulin resistance progresses and β cells are exhausted due to compensatory insulin hypersecretion, resulting in impaired insulin secretion and eventual β cell death) requiring insulin administration due to the destruction of pancreatic β cells, as well as for the suppression of the progression into complications.

In the agent for protecting and/or regenerating β cells (II) of the present invention, the HB-EGF expression vector of the present invention and the HGF expression vector of the present invention may be used as they are, or may be, where necessary, mixed with a pharmacologically acceptable carrier and formulated into various forms of preparation such as injection and the like, and used as pharmaceutical agents. When the nucleic acid encoding HB-EGF and the nucleic acid encoding HGF are inserted into separate expression vectors, the agent for protecting and/or regenerating β cells (II) of the present invention may be formulated as a single pharmaceutical composition containing the both expression vectors, or each expression vector may be separately formulated and used in combination. When each expression vector is separately formulated, a preparation containing an expression vector containing a nucleic acid encoding HB-EGF can be formulated in the same manner as in the aforementioned agent for protecting and/or regenerating β cells (I) of the present invention and administered by the same administration route. Also, when the nucleic acid encoding HB-EGF and the nucleic acid encoding HGF are inserted in a single expression vector, a preparation containing the expression vector can be formulated in the same manner as in the aforementioned agent for protecting and/or regenerating β cells (I) of the present invention and administered by the same administration route, dose, and administration frequency.

In the agent for protecting and/or regenerating β cells (II) of the present invention, when each expression vector is separately formulated, an expression vector containing a nucleic acid encoding HGF is formulated by blending various organic or inorganic carrier substances conventionally used as pharmaceutical preparation materials as solvents, solubilizing agents, suspending agents, isotonizing agents, buffering agents, soothing agents, and the like in liquid preparations. Also, as necessary, pharmaceutical preparation additives such as antiseptics, antioxidants, colorants, and the like can be used. As the solvents, suspending agents, isotonizing agents, buffering agents, soothing agents, antiseptics, antioxidants, and colorants, those respectively exemplified for the aforementioned agent for protecting and/or regenerating β cells (I) of the present invention can be preferably used in the same manner.

As examples of dosage forms of the aforementioned pharmaceutical composition, parenteral formulations such as injections (e.g., subcutaneous injections, intravenous injections, intramuscular injections, intraperitoneal injections, and the like), drip transfusion, and the like can be mentioned.

A preparation containing an expression vector containing a nucleic acid encoding HGF can be produced by a method conventionally used in the field of formulation technology, such as the method described in the Japanese Pharmacopoeia. The content of the viral vector, which is the active ingredient in the preparation, varies depending on the dosage form, dose of the active ingredient, and the like and is, for example, about 0.1 to 100 wt%. The virus titer can be appropriately adjusted to be about, for example, 10¹⁰ - 10¹¹ pfu/ml (since the physical titer is several to 100 times or more higher than the biological titer, 2×10¹⁰ - 2×10¹³ vp/ml in terms of virus particles), but is not limited to this range.

Examples of preparations suitable for parenteral administration (e.g., intravenous injection, subcutaneous injection, intramuscular injection, topical injection, intraperitoneal administration, and the like) include aqueous and nonaqueous isotonic aseptic injection liquids, in which antioxidant, buffering agent, antibacterial agent, isotonizing agent, and the like can be contained. The examples also include aqueous and nonaqueous aseptic suspension liquids optionally containing suspension agent, solubilizer, thickener, stabilizer, preservative, and the like. The most preferred dosage form of a preparation containing an expression vector containing a nucleic acid encoding HGF is an injection liquid.

When the agent for protecting and/or regenerating β cells (II) of the present invention is provided in a form in which each expression vector is separately formulated, the both preparations may be mixed at the time of use and administered as a single pharmaceutical composition, or may be administered as separate preparations simultaneously or in a staggered manner by the same or different administration routes.

The dose of the agent for protecting and/or regenerating β cells (II) of the present invention in which each expression vector is formulated separately varies depending on the kind of vector, promoter activity, administration route, severity of disease, species of animal to be the administration subject, and drug tolerance, body weight, age, and the like of the administration subject. For example, when Ad vectors are used as the HB-EGF expression vector and the HGF expression vector, for example, the total amount of the both expression vectors is about 5×10⁹ - about 5×10¹² vp/kg body weight, preferably about 1×10¹⁰ - about 2×10¹² vp/kg body weight, per one time. In addition, when AAV vectors are used as the HB-EGF expression vector and the HGF expression vector, for example, the total amount of the both expression vectors is about 5×10⁹ - about 5×10¹³ vp/kg body weight, preferably about 1×10¹⁰ - about 1×10¹³ vp/kg body weight, per one time.

On the other hand, when non-viral vectors encapsulated in liposomes are used as the HB-EGF expression vector and the HGF expression vector, for example, the total amount of the both expression vectors is about 2 to about 10 mg, preferably about 5 mg to about 8 mg, per one time for an adult.

The quantitative ratio of the both expression vectors in the agent for protecting and/or regenerating β cells (II) of the present invention, in which each expression vector is separately formulated, is not particularly limited as long as it provides the desired β-cell protective and/or regenerative effect (suppression of fasting and postprandial hyperglycemia, maintenance of glucose responsive insulin secretory capacity). For example, an HB-EGF expression vector:HGF expression vector ratio can be appropriately selected within the range of 10:1 - 1:10, preferably 5:1 - 1:5, more preferably 2:1 - 1:2.

The present invention is explained in more detail in the following by illustrating Examples. They are mere exemplifications and do not limit the scope of the present invention in any manner.

### [Example]

### Experiment method

### 1. Production of recombinant adenoviral vector (Ad)

Non-proliferating Ads expressing human HB-EGF under the transcriptional control of a hybrid promoter of the cytomegalovirus immediate early enhancer and a modified chicken β-actin promoter (CA promoter) or the Rous sarcoma virus (RSV) promoter (to be respectively referred to as "Ad.CA-HB-EGF", "Ad.RSV-HB-EGF" in the present specification), a non-proliferating Ad expressing human HGF under the transcriptional control of a CA promoter (to be respectively referred to as "Ad.CA-HGF" in the present specification), and a non-proliferating Ad expressing LacZ gene under the transcriptional control of a CA promoter (to be respectively referred to as "Ad.CA-LacZ" in the present specification) were produced by the method described in HUMAN GENE THERAPY 10:2013-2017 (1999). The recombinant Ads thus produced were proliferated and purified, and the titer was measured by conventional methods such as those described in Proc. Natl. Acad. Sci. USA 92: 2577-2581 (1995).

### 2. Animal experiment

Male 8-week-old c57/BL/6N mice (Kyudo Co., Ltd., Tosu) weighing 18 g-20 g were bred with free access to food and water. As previously reported (Diabetes 2010; 59: 1261-1265), streptozotocin (STZ; Sigma-Aldrich Japan, Tokyo) dissolved in 0.01 M citrate buffer (pH 4.5) was administered intraperitoneally to 40 mice at a dose of 50 mg/kg once daily for 5 consecutive days (days -7 to -3). STZ-injected mice were randomly divided into 4 groups (10 mice in each group) 3 days later (day 0), and any of
(1) 2×10¹¹ vp Ad.CA-LacZ
(2) 1×10¹¹ vp Ad.CA-HB-EGF+1×10¹¹ vp Ad.CA-LacZ
(3) 1×10¹¹ vp Ad.RSV-HB-EGF+1×10¹¹ vp Ad.CA-LacZ
(4) 1×10¹¹ vp Ad.CA-HB-EGF+1×10¹¹ vp Ad.CA-HGF
was injected once into the tail vein. Mice (n=4) that received neither STZ injection nor adenoviral gene therapy were used as normal controls (intact). Blood was collected from all mice, including those in the normal group. Blood samples were collected daily from day -7 to day 7, and once a week from day 14 to day 70. Blood glucose levels were tested at the time points indicated in Fig. 1. Intraperitoneal glucose tolerance test (IPGTT) was performed 16 and 60 days after Ad vector administration. After fasting the mice for 14 hr, 2 g/kg body weight of glucose (equivalent to 75 g of oral glucose tolerance test (OGTT) in humans) was administered intraperitoneally, and blood samples were collected immediately before administration, 30, 60 and 120 min after administration. Blood glucose level and plasma insulin level were measured at each time point. Insulin was measured using an ultrasensitive mouse insulin measurement ELISA kit (Morinaga Institute of Biological Science).

All animal experiments were conducted in accordance with the guidelines of the National Institutes of Health, and under an approval from the Kagoshima University Animal Experiment Ethics Committee.

### 3. Biochemical analysis

The blood glucose level was measured using Medisafefit Pro II (Terumo, Tokyo), and plasma insulin levels were measured by ELISA assay (Morinaga Co., Ltd., Yokohama).

Plasma aspartate aminotransferase (AST) and plasma alanine aminotransferase (ALT) levels were measured by SPOTCHEM SP-4430 clinical autometer (Arkray) using samples from 3, 5, 7, and 14 days after virus administration in each group.

### 4. Statistical analysis

Data are presented as mean±standard error (s.e.). Multiple comparisons were tested by one-way configuration dispersion analysis (One-way ANOVA), and the presence or absence of statistically significant differences between mice treated with Ad.CA-LacZ and mice treated with Ad.CA-HB-EGF+Ad.CA-HGF was determined by Student's t-test. P<0.05 was defined as statistically significant.

### 5. Histological analysis

Using the mouse liver after virus administration, a scientific analysis was performed in the same manner as described in Int J Mol Med.38(6):1673-1682 (2016) by using samples of each group 3, 5, 7, and 14 days after virus administration.

### Experiment results

### 1. Suppression of acute-phase hyperglycemia in T1D model mice by HB-EGF (and HGF) gene therapy with Ad vector

In the non-treated group (Ad.CA-LacZ administration group), the blood glucose concentration rapidly elevated to about 400 mg/dl on day 7 after vector administration (14 days after the first STZ injection) (Fig. 1A), gradually elevated thereafter, and then remained at a high level of not less than 400 mg/dl (Fig. 1B). A single intravenous injection of 1×10¹¹ vp Ad.CA-HB-EGF or Ad.RSV-HB-EGF suppressed an elevation in the blood glucose level by day 7 (Fig. 1A). In progress observation for about 10 weeks thereafter, the blood glucose level showed a tendency toward suppression as compared to the non-treated group, and a long-term effect was recognized (Fig. 1B). In the group with a combined use of the HB-EGF gene and the HGF gene, hyperglycemia was suppressed more strongly than in the case of the HB-EGF gene alone, and a significant effect was exhibited over a long period of time.

### 2. Suppression of postprandial hyperglycemia and glucose-responsive insulin secretion capacity in T1D model mice by HB-EGF (and HGF) gene therapy with Ad vector

IPGTT was performed 16 days and 60 days after administration of the therapeutic gene, and suppression of postprandial hyperglycemia and glucose-responsive insulin secretion capacity were confirmed. On the 16th day after administration in the HB-EGF gene administration group, suppression of elevation of blood glucose after glucose administration was observed (Fig. 2A), and an increase in insulin secretion amount was confirmed in response to glucose stimulation (Fig. 2B). These effects were synergistically increased by combining the HB-EGF gene with the HGF gene, similar to the blood glucose level. Similar results were observed 60 days after administration (Figs. 3A, 3B). From the above, it was clarified that gene therapy improves glucose tolerance over a long term.

### 3. Intravenous administration of Ad vector of this Example does not cause liver dysfunction

Preclinical and clinical studies have reported that intravenous administration of high doses of Ad vectors in vivo results in gene transfer primarily into the liver, which may lead to severe hepatopathy (Mol Genet Metab 2003; 80: 148-158.; Hum Gene Ther 2020; 31: 695-696). Therefore, in order to evaluate hepatopathy due to intravenous administration of the Ad vector of this Example, plasma AST and ALT levels were measured on days 3, 5, 7, and 14 in the same five groups of mice as in above-mentioned 1. (Fig. 4). No biochemical test (AST and ALT) showed abnormal values exceeding 200 IU/L (Fig. 4). Histological analysis of the Ad.CA-LacZ group showed that the degree of liver damage was mild to moderate (Fig. 5, Fig. 6), confirming that the dosage poses no safety problems. In addition, the HB-EGF group suppressed an increase of AST and ALT values as compared with the LacZ group (Fig. 4), and the HB-EGF/HGF group exhibited a strong suppressive effect (Figs. 1 - 3). From this, it was confirmed that HB-EGF, further HB-EGF/HGF, not only protect and/or regenerate β-cells in diabetes, but also suppress complication of liver dysfunction caused by virus administration.

### [Industrial Applicability]

HB-EGF gene therapy by systemic administration exhibits a protective and/or regenerative effect on pancreatic β cells, and exhibits a hyperglycemia-suppressing effect while maintaining normal insulin secretory capacity. Furthermore, by combining the HB-EGF gene with the HGF gene, the glucose-responsive insulin secretion capacity is markedly improved, and not only the elevation of random blood sugar and fasting blood sugar but also postprandial hyperglycemia can be markedly suppressed. Gene therapy using HB-EGF for diabetes by retrograde pancreatic duct injection, which is highly invasive and requires complicated techniques, has been reported so far. However, β cell differentiation and proliferation have been demonstrated only in normal mice, and the hyperglycemia suppressive effect has not been sufficiently demonstrated. According to the present invention, systemic administration such as intravenous injection, and even administration at a low dose equivalent to pancreatic local administration, exhibits superior β-cell protecting and/or regenerating effects and hyperglycemia-suppressing effects. Furthermore, even when a chromosomally non-integrating virus vector, which has conventionally been considered relatively safe, is used, the effect is maintained for a long period of time far exceeding the expected gene expression period. Therefore, the agent for protecting and/or regenerating β cells of the present invention can be a safe and effective gene therapeutic agent for diabetes that can be clinically applied. The number of diabetes patients continues to increase worldwide and has become a social problem, and the significance of the present invention is great. In particular, T1D develops at a young age, and existing pancreatic islet transplantation therapy is limited in its use. Therefore, the agent for protecting and/or regenerating β cells of the present invention is extremely useful as an alternative and versatile means of regenerative medicine for diabetes including T1D.

This application is based on a patent application No. 2021-033714 filed in Japan (filing date: March 3, 2021) and a patent application No. 2021-145796 filed in Japan (filing date: September 7, 2021), the contents of which are incorporated in full herein.

## Claims

1. An agent for protecting and/or regenerating a pancreatic β cell in a mammal with diabetes, comprising a nucleic acid encoding a heparin-binding epidermal growth factor-like growth factor (HB-EGF), wherein the agent is systemically administered.

2. The agent according to claim 1, in combination with a nucleic acid encoding hepatocyte growth factor (HGF).

3. The agent according to claim 1 or 2, wherein the pancreatic β cell retains glucose-responsive insulin secretory capacity.

4. The agent according to any one of claims 1 to 3, wherein the systemic administration is intravenous administration.

5. The agent according to any one of claims 1 to 4, wherein the nucleic acid is carried on a viral vector.

6. The agent according to claim 5, wherein the viral vector is an adenovirus (Ad) vector or an adeno-associated virus (AAV) vector.

7. The agent according to claim 6, wherein the agent is administered in a single dose or administered in multiple doses with at least 60 days interval.

8. The agent according to any one of claims 5 to 7, wherein the viral vector is administered at a dose of 1×10¹⁰ to 2×10¹² viral particles (vp)/kg body weight.

9. The agent according to any one of claims 1 to 8, wherein the diabetes is type 1 diabetes.

10. The agent according to any one of claims 1 to 9, wherein the mammal is human.

11. A method of protecting and/or regenerating a pancreatic β cell in a mammal with diabetes comprising systemically administering to the mammal an effective amount of a nucleic acid encoding HB-EGF.

12. The method according to claim 11, further comprising administering to the mammal an effective amount of a nucleic acid encoding HGF.
